# EUROPÄISCHE PATENTSCHRIFT

(11) **EP 3 694 451 B1**
(45) Veröffentlichungstag und Bekanntmachung des Hinweises auf die Patenterteilung: **03.12.2025**
(21) Anmeldenummer: 18785588.7
(22) Anmeldetag: 09.10.2018
(51) Int. Cl.: A61F 5/02

(54) **VORRICHTUNG ZUR UNTERSTÜTZUNG VON BEWEGUNGEN UND HUBBEWEGUNGEN DES MENSCHLICHEN KÖRPERS**
DEVICE FOR SUPPORTING MOVEMENT AND LIFTING MOVEMENTS OF A HUMAN BODY
DISPOSITIF DE SUPPORT POUR MOUVEMENTS ET MOUVEMENTS DE LEVER D'UN CORPS HUMAIN

(30) Priorität: 10.10.2017 DE 102017123518
(43) Veröffentlichungstag der Anmeldung: 19.08.2020
(73) Patentinhaber: HUNIC GmbH, 72270 Baiersbronn (DE)
(72) Erfinder: MAST, Jonas, 72270 Baiersbronn (DE)
(74) Vertreter: Westphal, Mussgnug & Partner Patentanwälte mbB
(86) Internationale Anmeldenummer: PCT/EP2018/077492
(87) Internationale Veröffentlichungsnummer: WO 2019/072855

(56) Entgegenhaltungen:
- US-A- 6 099 446
- US-A1- 2010 121 240
- US-B1- 6 190 342
- US-B1- 6 450 131
- US-B2- 7 662 121

## Beschreibung

Die Erfindung betrifft eine Vorrichtung zur Unterstützung von Bewegungen und Hubbewegungen des menschlichen Körpers, welche am Körper tragbar ist.

Viele Personen, insbesondere gewerbliche Arbeitnehmer, sind bei unterschiedlichen Tätigkeiten hohen physischen Belastungen ausgesetzt. Hierbei wird insbesondere der Rücken beim Heben und Tragen von Lasen, durch ungünstige Haltungen oder sich ständig wiederholende manuelle Tätigkeiten stark belastet. Um hier eine Unterstützung zu ermöglichen, sind Vorrichtungen zur Unterstützung von Bewegungen und Hubbewegungen des menschlichen Körpers, welche am Körper tragbar sind, bekannt. Derartige Vorrichtungen werden auch als Exoskelette bezeichnet.

Dabei sind aktive und passive Exoskelette bekannt. Aktive Exoskelette zeichnen sich dadurch aus, dass sie elektrische Motoren, hydraulische Komponenten oder ähnliche Systeme aufweisen, die Bewegungen der Gelenke des menschlichen Körpers durch ihren Antrieb aktiv unterstützen können. Die Person erfährt somit eine direkte Unterstützung bei seinen Bewegungen und Tätigkeiten. Aktive Exoskelette weisen aufgrund der Maschinen Elemente jedoch ein hohes Eigengewicht auf. Zudem ist die Abstimmung des Antriebs der Maschinenelemente auf den Bewegungsvorgang der Person nicht einfach, da die Maschinenelemente erkennen müssen, wann welcher Bewegungsvorgang durchgeführt wird. Schließlich tragen derartige aktive Exoskelette vom Bauraum her auf und sind unhandlich zu handhaben.

Passive Exoskelette weisen keinen Antrieb auf, sondern speichern potentielle Energie bei einer Flexion des Körpers oder des entsprechenden Gelenks. Bei einer Reflexion wird diese Energie wieder freigegeben und unterstützt die entsprechende Bewegung. Bekannt ist beispielsweise die Verwendung von Federn. Das Eigengewicht von passiven Exoskeletten ist in der Regel geringer als das von aktiven Exoskeletten, und üblicherweise trägt das passive Exoskelett weniger auf als das aktive Exoskelett.

Als Stand der Technik werden die US 6 190 342 B1, die US 6 099 446 A, die US 7 662 121 B2, die US 2010/121 240 A1 sowie die US 6 450 131 B1 genannt.

Die Aufgabe der Erfindung besteht darin, eine Vorrichtung zur Unterstützung von Bewegungen und Hubbewegungen des menschlichen Körpers, insbesondere ein passives Exoskelett, derart weiterzubilden, dass es mehrere Funktionalitäten aufweist. Insbesondere ist wünschenswert, eine derartige Vorrichtung bereitzustellen, welche einerseits eine unterstützende Funktion von Bewegungen, insbesondere Hebebewegungen, andererseits aber auch eine präventive Funktion, insbesondere durch Einschränkung von gesundheitsschädlichen Bewegungen, aufweist.

Die Aufgabe wird erfindungsgemäß gelöst durch eine Vorrichtung zur Unterstützung von Bewegungen und Hubbewegungen des menschlichen Körpers, insbesondere ein passives Exoskelett, mit den Merkmalen des Patentanspruchs 1.

Vorteilhafte Ausgestaltungen und Weiterbildungen der Erfindung sind in den abhängigen Ansprüchen angegeben.

Die erfindungsgemäße Vorrichtung zur Unterstützung von Bewegungen und Hebebewegungen des menschlichen Körpers, insbesondere das erfindungsgemäße passive Exoskelett, welche am Körper tragbar ist, weist ein Hüftelement, zwei Schulterbänder, ein Rückenelement, welches an dem Hüftelement angeordnet ist und zumindest ein Versteifungselement aufweist, das zumindest abschnittsweise aus einem formelastischen Material gefertigt ist, und zwei an dem Hüftelement angeordnete Beinbänder auf, die zumindest abschnittsweise aus einem Elastomer gefertigt sind. Dabei sind die Beinbänder so an dem Hüftelement angeordnet, dass sie in Verlängerung des Rückenelements verlaufen, und weisen jeweils ein Knieelement auf, welches eine obere Knieschlaufe, eine untere Knieschlaufe und ein die Kniescheibe freilassendes Kniescheibenelement auf.

Als Hüftelement soll im Folgenden ein Element verstanden werden, das im an eine Person angelegten Zustand der Vorrichtung im Bereich der Hüfte an der Person anliegt. Es kann, muss aber nicht zwingenderweise, zur Befestigung der Vorrichtung an der Person ausgelegt sein.

Als Rückenelement soll im Folgenden ein Element verstanden werden, dass zumindest abschnittsweise im an eine Person angelegten Zustand der Vorrichtung am Rücken der Person anliegt. Das Rückenelement kann einstückig mit dem Hüftelement verbunden sein.

Als Schulterbänder werden im Folgenden die Elemente verstanden, die das Rückenelement im an die Person angelegten Zustand der Vorrichtung am Rücken der Person anliegend fixieren. Sie können vom Rücken über die Schulter auf die Brust der Person und unterhalb der Arm wieder zum Rücken geführt verlaufen. Es ist aber auch möglich, dass das Rückenelement sowohl am Rücken als auch auf den Schultern aufliegt und die Schulterbänder nicht auf der Schulter selbst, sondern im Wesentlichen auf der Brust der Person verlaufen.

Die erfindungsgemäße Vorrichtung zeichnet sich somit dadurch aus, dass sie einerseits zumindest abschnittsweise elastische Komponenten aufweist, welche bei Flexion der Gelenke Energie aufnehmen und diese bei Reflexion wieder freisetzen, um auf diese Weise die entsprechende Bewegung der Person zu unterstützen, andererseits jedoch auch zumindest abschnittsweise formelastische oder mit anderen Worten quasi starre oder quasi steife Komponenten aufweist, die gesundheitsschädliche Bewegungen erschweren oder gar unterbinden.

Beispielsweise können beim Hinhocken oder beim Vorbeugen die aus einem Elastomer gefertigten Beinbänder, welche in Verlängerung des Rückenelements verlaufen, insbesondere durch den verlängerten Weg über das Gesäß der Person gespannt werden, wobei beim Aufstehen oder Aufrichten die Energie der Elastomere wieder freigesetzt werden und dadurch das Aufstehen oder Aufrichten der Person unterstützt werden kann. Allerdings kann beispielsweise ein gesundheitsschädliches Beugen aus der Wirbelsäule aufgrund des Versteifungselements nahezu unterbunden werden, wenn das Versteifungselement nur ein Vorbeugen mit geradem Rücken zulässt, wodurch die Bandscheiben entlastet werden können. Wenn das Versteifungselement die Torsion des Oberkörpers einschränkt, kann eine Passion unter Last vermieden werden und die Person beispielsweise dazu angehalten werden, die relative Position zwischen Standpunkt und der zu hebenden oder bewegenden Last zu ändern.

Das erfindungsgemäße Knieelement unterstützt das Kniegelenk, wobei die die Kniescheibe freilassende Ausgestaltung einen größeren Bewegungsraum der Kniescheibe ermöglicht und den Druck auf die Kniescheibe verringert. Zudem verlaufen die Beinbänder bei der erfindungsgemäßen Anordnung vom Rücken nach vorne über das Knie, um eine Rückstellkraft erzeugen zu können.

Insgesamt bietet somit die erfindungsgemäße Vorrichtung einen Vorteil sowohl hinsichtlich der Unterstützungsfunktion als auch der Prävention auf. Die erfindungsgemäße Vorrichtung kann dabei gleichzeitig den Vorteil aufweisen, dass bei Verwendung von sehr leichten Materialien ein geringes Eigengewicht erzielt werden kann. Die Vorrichtung kann einen sehr geringen Bauraum aufweisen und kann dadurch sowohl über der Kleidung als auch unter der Kleidung getragen werden.

Gemäß einer vorteilhaften Weiterbildung der Erfindung ist das Versteifungselement im an den Körper angelegten Zustand der Vorrichtung im Wesentlichen parallel zur Wirbelsäule ausgerichtet. Ein derart angeordnetes Versteifungselement kann der Flexion des Rückens über einen gewissen Winkel hinaus, der Torsion des Oberkörpers über einen gewissen Winkel hinaus und/oder der seitlichen Neigebewegungen über einen gewissen Winkel hinaus, entgegenwirken.

Der gewünschte Effekt der Versteifung kann dadurch verbessert werden, dass das Rückenelement zwei oder mehr Versteifungselemente aufweist.

Um das Eigengewicht der erfindungsgemäßen Vorrichtung möglichst gering zu halten, ist bei einer vorteilhaften Ausgestaltung das Versteifungselement aus Kunststoff gefertigt.

Gemäß einer besonders bevorzugten Ausführungsform der Erfindung weist das formelastische Material ein E-Modul von mehr als 600 N/mm², beispielsweise ein E-Modul von mehr als 1000 N/mm², insbesondere ein E-Modul von etwa 5000 N/mm², auf. Derartige Materialien weisen eine ausreichende Steifigkeit auf, um den gesundheitsschädigenden Bewegungen entgegenwirken zu können. Derartige Materialien können je nach E-Modul gleichzeitig aber noch eine geringe Elastizität aufweisen, um ausreichend Tragekomfort bieten zu können.

Eine besonders bevorzugte Ausführungsform der Erfindung sieht vor, dass das Versteifungselement auswechselbar an dem Rückenelement angeordnet ist, vorzugsweise in eine an dem Rückenelement angeordnete Tasche eingeschoben ist. Dies ermöglicht einerseits eine Anpassung der Art des Versteifungselements an die jeweilige Person. Andererseits kann die Reinigbarkeit der Vorrichtung verbessert werden.

Um die Vorrichtung auf einfache Art und Weise an die Größe der Person anpassen zu können, sind die Schulterbänder in ihrer Länge vorteilhafterweise verstellbar ausgebildet.

Vorzugsweise sind die Schulterbänder jeweils mit einem Ende an dem Rückenelement und mit dem anderen Ende entweder an dem Rückenelement oder dem Hüftelement angeordnet, was einen einfachen Aufbau der Vorrichtung ermöglicht.

Gemäß einer vorteilhaften Ausführungsform ist das Hüftelement als Hüftgurt ausgebildet, wodurch eine zuverlässige Befestigung der Vorrichtung an einer Person ermöglicht werden kann. Der Hüftgurt ist vorzugsweise in der Weite verstellbar, so dass eine Anpassung an den Hüftumfang der Person, welche die Vorrichtung anlegt, erfolgen kann.

Gemäß einer vorteilhaften Ausgestaltung der Erfindung weist das Elastomer ein E-Modul von weniger als 600 N/mm², beispielsweise von weniger als 150 N/mm², insbesondere von weniger als 100 N/mm², beispielsweise ein E-Modul von etwa 30 N/mm², auf. Dies ermöglicht ausreichend Energie bei der Flexion aufzunehmen und bei der Reflexion ausreichende Unterstützung bieten zu können.

Vorteilhafterweise ist jedes der Beinbänder lösbar an dem Hüftelement befestigt. Dabei können die Beinbänder jeweils einzeln lösbar an dem Hüftelement befestigt sein oder auch über ein gemeinsames Befestigungselement lösbar an dem Hüftelement befestigt sein. Dies ermöglicht ein einfacheres Anlegen der Vorrichtung. Dabei sind die Beinbändern insbesondere derart an dem Hüftelement befestigt, dass sie im an die Person angelegten Zustand der Vorrichtung über das Gesäß der Person an die Rückseite der Beine der Person geführt verlaufen. Insbesondere verlaufen die Beinbänder im an die Person angelegten Zustand der Vorrichtung etwa parallel zur Längsachse des Körpers.

Eine bevorzugte Ausführungsform der Erfindung sieht vor, dass jedes der Beinbänder in der Länge verstellbar ausgebildet ist, wodurch die Vorrichtung auf einfache Art und Weise an die Größe der Person, die die Vorrichtung anlegt, angepasst werden kann.

Eine vorteilhafte Weiterbildung der Erfindung sieht vor, dass die obere Knieschlaufe und/oder die untere Knieschlaufe öffenbar und wiederverschließbar, vorzugsweise mittels eines Klettverschlusses oder eines sonstigen Verschlusses, ausgebildet sind. Dies vereinfacht das Anlegen des Knieelements, da ein Überstreifen der Knieschlaufe ausgehend vom Fuß bis zum Kniebereich vermieden werden kann.

Vorteilhafterweise weist jedes der Beinbänder ein Fußelement auf, welches eine Knöchelschlaufe und eine Sohlenschlaufe aufweist, welche insbesondere derart miteinander verbunden sind, dass sie ein 8-förmiges Element bilden. Ein derartiges Fußelement ermöglicht eine zuverlässige Fixierung des Beinbands am jeweiligen Fuß. Zudem ermöglicht ein derartiges Fußelement eine gute Kraftableitung über die Fußsohle. Das Fußelement ist vorzugsweise lösbar befestigt, was das Anlegen der Vorrichtung vereinfachen kann und was ein Auswechseln der Fußelemente ermöglicht.

Eine bevorzugte Weiterbildung der Erfindung sieht vor, dass zwischen der Knöchelschlaufe und der Sohlenschlaufe ein Fersenband angeordnet ist, welches vorzugsweise an ihren einander abgewandten Bereichen befestigt ist. Ein derartiges Fersenband kann einem Verrutschen des Fußelement entgegenwirken.

Vorzugsweise sind die Beinbänder in dem Bereich zwischen dem Knieelement und dem Fußelement in ihrer Länge verstellbar ausgebildet, um die Länge der Beinbänder an die Größe einer die Vorrichtung tragenden Person anpassen zu können.

Die Erfindung wird anhand der nachfolgenden Figuren ausführlich erläutert. Es zeigen:
- Fig. 1: eine Ansicht von vorne auf ein Ausführungsbeispiel einer erfindungsgemäßen Vorrichtung,
- Fig. 2: eine Ansicht von hinten auf die Vorrichtung gemäß Fig. 1,
- Fig. 3: eine Ansicht von vorne auf die Vorrichtung gemäß Fig. 1 im an eine Person angelegten Zustand,
- Fig. 4: eine Ansicht von hinten auf die Vorrichtung gemäß Fig. 1 im an eine Person angelegten Zustand,
- Fig. 5: ein Ansicht von vorne auf ein alternatives Ausführungsbeispiel eines Fußelements einer Vorrichtung gemäß Fig. 1,
- Fig. 6: eine Seitenansicht des Fußelements gemäß Fig. 5 und
- Fig. 7: eine Ansicht von hinten auf ein zweites Ausführungsbeispiel einer erfindungsgemäßen Vorrichtung.

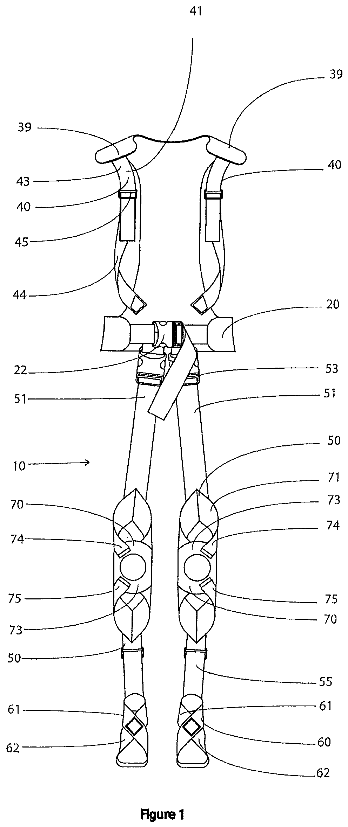

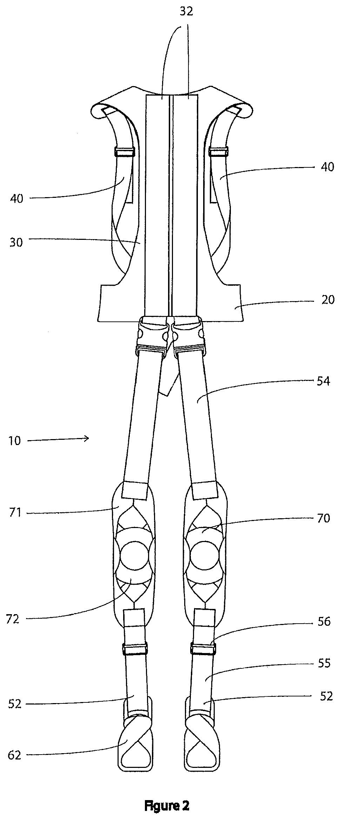

Die Figuren 1 bis 4 zeigen verschiedene Ansichten eines Ausführungsbeispiels einer Vorrichtung 10 zur Unterstützung von Bewegungen und Hebebewegungen des menschlichen Körpers, insbesondere eines passiven Exoskeletts. Zur besseren Übersicht sind nicht sämtliche Bezugsziffern in sämtlichen Figuren angegeben.

Die Vorrichtung 10 weist ein Hüftelement 20 auf, welches in der vorliegenden Ausführungsform als Hüftgurt ausgebildet ist. Das Hüftelement 20 kann im Bereich der Hüfte an einer Person fixiert werden, beispielsweise, indem es die Hüfte umgreift. Zur Vereinfachung des Anlegens kann das Hüftelement eine Schnappschnalle 22 oder alternativ auch einen Verschluss nach Art eines Gürtels oder einen Klettverschluss aufweisen. Dabei ist insbesondere wenigstens ein Ende des Hüftgurts längenverstellbar an der Schnappschnalle 22 angeordnet, um die Weite des Hüftelements 20 verstellen zu können.

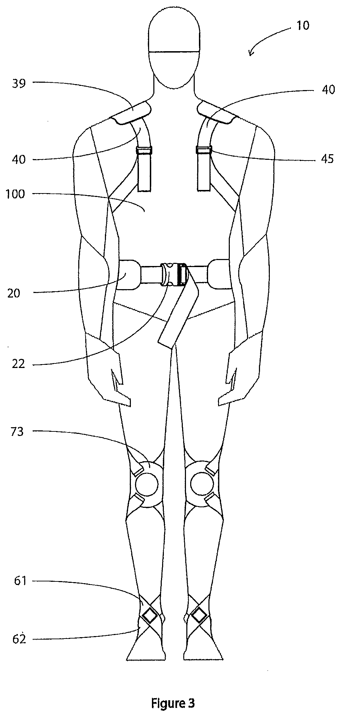

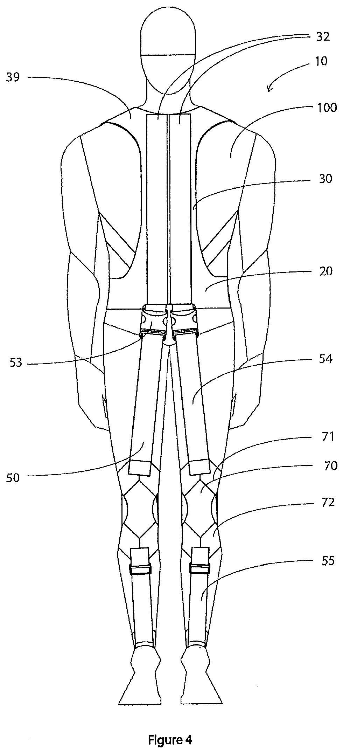

An dem Hüftelement 20 ist vorzugsweise einstückig ein Rückenelement 30 angeordnet, welches insbesondere eine derartige Länge aufweist, dass es im an eine Person 100 angelegten Zustand (vgl. Fig. 3 und 4) vom Bereich der Hüfte bis in den Bereich des oberen Rückens oder Nackens erstreckt. Insbesondere kann das Rückenelement 30 auch an seinem dem Hüftelement 20 abgewandten Ende mit Abschnitten 39 auf den Schultern der Person 100 aufliegen.

Das Hüftelement 20 und das Rückenelement 30 können aus einem textilen Material gefertigt sein.

Die Vorrichtung 10 weist zwei Schulterbänder 40 auf, welche jeweils ein erstens Ende 41 und ein zweites Ende 42 aufweisen. Das erste Ende 41 ist an dem Rückenelement 30, beispielsweise an den auf den Schultern der Person 100 aufliegenden abschnitten 39 des Rückenelements 30, angeordnet. Das zweite Ende 42 kann an dem Hüftelement 20 oder dem Rückenelement 30 angeordnet sein und ist im vorliegenden Ausführungsbeispiel am Rückenelement 30 im Bereich des dem Hüftgurt 20 zugewandten Endes angeordnet. Die Schulterbänder 40 können zweigeteilt in zwei Abschnitte 43, 44 ausgebildet sein, wobei der eine Abschnitt 43 das erste Ende 41 und der andere Abschnitt 44 das zweite Ende 42 umfasst, und die beiden Abschnitte 43, 44 über eine Schnalle 45 miteinander verbunden sind. Die Schnalle 45 erlaubt insbesondere eine Längenverstellbarkeit des Schulterbands 40. Die Schulterbänder 40 können aus einem Gurtmaterial, beispielsweise aus einem Gewebeband, gefertigt sein.

Die Vorrichtung 10 kann von der Person 100 angelegt werden, indem die Arme durch die durch das Rückenelement 30 und die Schulterbänder 40 gebildeten Schlaufen geführt werden, bis das Rückenelement 30 am Rücken anliegt, und anschließend der Hüftgurt geschlossen wird. Dabei kann mithilfe der Schnallen 45 die gewünschte Länge der Schulterbänder 40 und mithilfe der Schnappschnalle 22 die gewünschte Weite des Hüftelements 20 eingestellt werden, bis die Vorrichtung 10 eine Position erreicht hat, in welcher sie am Körper anliegt.

Das Rückenelement 30 weist zumindest ein Versteifungselement 32, beispielsweise zwei Versteifungselemente 32 auf, welche insbesondere parallel zueinander und vorzugsweise im an die Person 100 angelegten Zustand der Vorrichtung 10 etwa parallel zur Wirbelsäule verlaufend ausgerichtet sind. Die Versteifungselemente 32 können an der Außenseite des Rückenelements 30 befestigt oder in das Rückenelement 30 integriert sein. Alternativ können die Versteifungselemente 32 auch in an dem Rückenelement 30 ausgebildete Taschen eingeschoben und damit insbesondere auswechselbar ausgebildet sein.

Das Versteifungselement 32 ist zumindest abschnittsweise, vorzugsweise vollständig, aus einem formelastischen Material gefertigt. Das formelastische Material kann beispielsweise Metall oder Kunststoff sein, wobei Kunststoff aufgrund des geringeren Gewichts in der Regel bevorzugt wird. Das formelastische Material weist vorzugsweise ein E-Modul von mehr als 600 N/mm², besonders bevorzugt ein E-Modul von mehr als 1000 N/mm², beispielsweise ein E-Modul von 5000 N/mm² auf.

An dem Hüftelement 20 sind zwei Beinbänder 50 angeordnet, insbesondere derart, dass am in Verlängerung des Rückenelements 30 verlaufen, oder anders ausgedrückt insbesondere derart, dass sie im an die Person 100 angelegten Zustand der Vorrichtung ausgehend von der Hüfte über das Gesäß der Person 100 an den Beinen anliegend verlaufen. Die Beinbänder 50 weisen ein ersten Ende 51 und ein zweites Ende 52 auf. Die Beinbänder 50 sind vorzugsweise lösbar mit dem ersten Ende 51 an dem Hüftelement 20 angeordnet, beispielsweise mittels einer Schnappschnalle 53 oder alternativ nach Art eines Verschluss eines Gürtels oder mittels eines Klettverschlusses oder sonstigen Verschlusses. Dabei ist das Beinband 50 vorzugsweise derart an der Schnappschnalle 53 oder dem sonstigen Verschluss befestigt, dass eine Längenverstellbarkeit des Beinbands 50 ermöglicht wird.

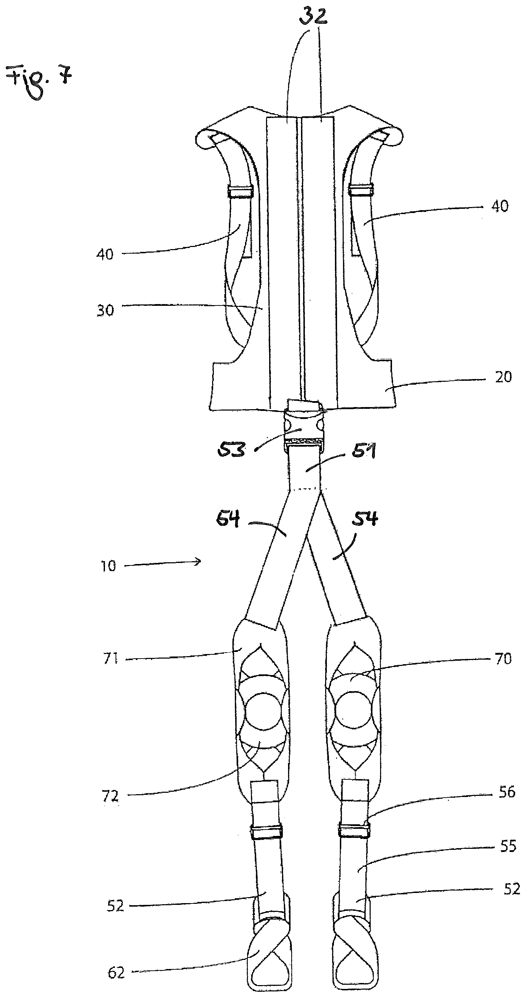

Dabei sind die Beinbänder 50 entweder wie im ersten in den Figuren 1 bis 4 dargestellten Ausführungsbeispiel jeweils einzeln mit dem ersten Ende 51 an dem Hüftelement 20 lösbar angeordnet und können jeweils einzeln gelöst oder befestigt werden. Alternativ können die Beinbänder 50 wie in einem zweiten anhand von Figur 7 dargestellten Ausführungsbeispiel, welches sich nur in der Befestigung der Beinbänder 50 an dem Hüftelement 20 von dem in den Figuren 1 bis 4 dargestellten Ausführungsbeispiel unterscheidet, mit ihren ersten Enden 51 gemeinsam an dem Hüftelement 20 angeordnet sein und gemeinsam lösbar befestigt sein, beispielsweise mittels einer einzigen Schnappschnalle 53 oder alternativ nach Art eines Verschluss eines Gürtels oder mittels eines Klettverschlusses oder sonstigen Verschlusses.

An dem zweiten Ende 52 des Beinbands 50 ist vorzugsweise ein Fußelement 60 zur Befestigung des zweiten Endes 52 des Beinbands 50 an einem Fuß der Person 100 angeordnet. Das Fußelement 60 kann lösbar an dem Beinband 50 befestigt sein, beispielsweise mittels eines Klettverschlusses. Das Fußelement 60 kann eine Knöchelschlaufe 61 und eine Sohlenschlaufe 62 aufweisen, welche insbesondere derart miteinander verbunden sind, dass sie ein 8-förmiges Element bilden. Das Fußelement 60 wird derart über den Fuß der Person 100 gestreift, dass die Knöchelschlaufe 61 den Knöchel umschließt und die Sohlenschlaufe 62 über den Fußrücken und die Fußsohle verlaufend angeordnet ist.

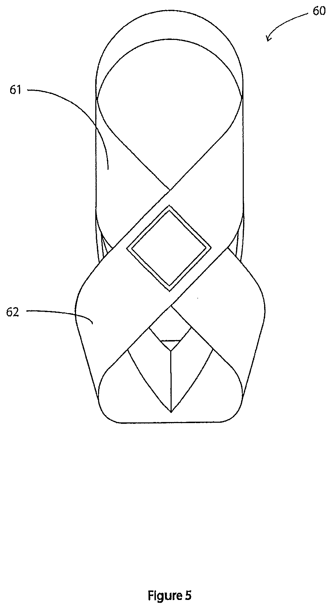

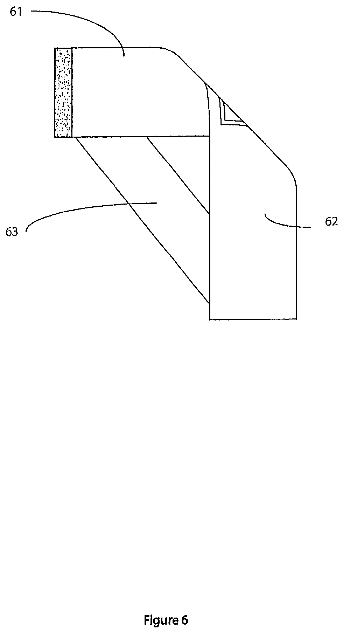

Eine alternative Ausführungsform des Fußelements 60 ist in den Figuren 5 und 6 dargestellt, wobei sich das dort dargestellte Ausführungsbeispiel des Fußelements 60 von dem in den Figuren 1 bis 4 dargestellten Ausführungsbeispiel des Fußelements 60 nur durch ein zusätzliches Fersenband 63 unterscheidet. Das Fersenband 63 ist insbesondere derart angeordnet, dass es im an die Person 100 angelegten Zustand des Fußelements 60 über die Ferse bis zur Fußsohle verläuft. Das Fersenband 63 verbindet vorzugsweise die einander abgewandten Enden der Knöchelschlaufe 61 und der Sohlenschlaufe 62.

Das Beinband 50 kann ein Knieelement 70 aufweisen. Das Knieelement 70 kann eine obere Knieschlaufe 71, eine untere Knieschlaufe 72 und ein Kniescheibenelement 73 aufweisen, wobei das Kniescheibenelement 73 vorzugsweise derart ausgebildet ist, dass es ein Loch aufweist und im an die Person 100 angelegten Zustand der Vorrichtung 10 die Kniescheibe umfassend ausgebildet ist. Im an die Person 100 angelegten Zustand der Vorrichtung 100 umgreift die obere Knieschlaufe 71 das Bein der Person 100 oberhalb des Knies, insbesondere im unteren Bereich des Oberschenkels. Die untere Knieschlaufe 72 umgreift im an die Person 100 angelegten Zustand der Vorrichtung 100 das Bein der Person 100 unterhalb des Knies, insbesondere im oberen Bereich des Unterschenkels. Das Kniescheibenelement 73 umgreift dabei die Kniescheibe und lässt diese dabei vorzugsweise frei.

Das Beinband 50 kann zweigeteilt mit einem ersten Abschnitt 54, welcher das erste Ende 51 umfasst, und einem zweiten Abschnitt 55, welcher das zweite Ende 52 umfasst ausgebildet sein, wobei vorzugsweise das Knieelement 70 zwischen dem ersten Abschnitt 54 und dem zweiten Abschnitt 55 ausgebildet ist. Der zweite Abschnitt 55 kann längenverstellbar ausgebildet sein. Dazu kann der zweite Abschnitt 55 zweigeteilt ausgebildet und die beiden Teile beispielsweise mithilfe einer Schnalle 56 verbunden sein.

Die Beinbänder 50 sind zumindest abschnittsweise aus einem Elastomer gefertigt. Vorzugsweise sind der erste Abschnitt 54 und/oder der zweite Abschnitt 55 aus einem Elastomer gefertigt. Auch das Knieelement 70 und/oder das Fußelement können aus einem Elastomer gefertigt sein. Das Elastomer kann insbesondere ein E-Modul von weniger als 600 N/mm², bevorzugt ein E-Modul von weniger als 150 N/mm², besonders bevorzugt ein E-Modul von weniger als 100 N/mm², vorzugsweise ein E-Modul von etwa 30 N/mm² aufweisen.

Die Beinbänder 50 können von der Person 100 derart angelegt werden, dass das Knieelement 70 und das Fußelement 60 unabhängig voneinander angelegt werden. Zum Anlegen des Fußelements 60 wird der Fuß mit den Zehen voran zunächst durch die Knöchelschlaufe 61 und anschließend durch die Sohlenschlaufe 62 geführt, bis die Knöchelschlaufe 61 um den Knöchel anliegt und die Sohlenschlaufe 62 über Fußrücken und Fußsohle verlaufend liegt, wobei der Verbindungsbereich zwischen Knöchelschlaufe 61 und Fußschlaufe 62 auf dem Fußrücken zu liegen kommt. Das Knieelement 70 wird in einer Ausführungsform derart über den Fuß das Bein hinauf gestreift, bis die obere Knieschlaufe 71 im unteren Bereich des Oberschenkels und die untere Knieschlaufe 72 im oberen Bereich des Unterschenkels anliegt. Alternativ ist es möglich, dass die obere Knieschlaufe 71 und/oder die untere Knieschlaufe 72 öffenbar und wiederverschließbar ausgebildet sind, beispielsweise mittels eines Klettverschlusses oder eines sonstigen Verschlusses 74, 75, wodurch ein Anlagen ohne Überstreifen ausgehend vom Fuß ermöglicht wird. Anschließend kann der der zweite Abschnitt 55 an dem Fußelement 60 befestigt werden und vorzugsweise die Länge des Abschnitts 55 an die Größe der Person 100 angepasst werden. Schließlich wird das Beinband 50 am Hüftelement 20 befestigt, beispielsweise mithilfe der Schnappschnalle 53, wobei vorzugsweise die Länge des Beinbands 50 auf die Größe der Person 100 abgestimmt wird.

### Bezugszeichenliste

- 10: Vorrichtung
- 20: Hüftelement
- 22: Schnappschnalle
- 30: Rückenelement
- 32: Versteifungselement
- 40: Schulterband
- 41: erstes Ende
- 42: zweites Ende
- 43: Abschnitt
- 44: Abschnitt
- 45: Schnalle
- 50: Beinband
- 51: erstes Ende
- 52: zweites Ende
- 53: Schnappschnalle
- 54: Abschnitt
- 55: Abschnitt
- 60: Fußelement
- 61: Knöchelschlaufe
- 62: Sohlenschlaufe
- 63: Fersenband
- 70: Knieelement
- 71: obere Knieschlaufe
- 72: untere Knieschlaufe
- 73: Kniescheibenelement
- 74: Verschluss
- 75: Verschluss
- 100: Person

## Patentansprüche

1. Vorrichtung (10) zur Unterstützung von Bewegungen und Hebebewegungen des menschlichen Körpers, welche am Körper tragbar ist, mit einem Hüftelement (20), mit zwei Schulterbändern (40), mit einem Rückenelement (30), welches an dem Hüftelement (20) angeordnet ist und zumindest ein Versteifungselement (32) aufweist, das zumindest abschnittsweise aus einem formelastischen Material gefertigt ist, und mit zwei an dem Hüftelement (20) angeordneten Beinbändern (50), die zumindest abschnittsweise aus einem Elastomer gefertigt sind und so an dem Hüftelement (20) angeordnet sind, dass sie in Verlängerung des Rückenelements (30) verlaufen, wobei jedes der Beinbänder (50) ein Knieelement (70) aufweist, welches eine obere Knieschlaufe (71), eine untere Knieschlaufe (72) und ein vorzugsweise die Kniescheibe freilassende Kniescheibenelement (73) aufweist,
**dadurch gekennzeichnet, dass** die Beinbänder vom Rücken nach vorne über das Knie verlaufen.

2. Vorrichtung nach Anspruch 1,
**dadurch gekennzeichnet, dass** das Versteifungselement (32) im an den Körper angelegten Zustand der Vorrichtung (10) im Wesentlichen parallel zur Wirbelsäule ausgerichtet ist.

3. Vorrichtung nach einem der vorhergehenden Ansprüche, **dadurch gekennzeichnet, dass** das Rückenelement (30) zwei oder mehr Versteifungselemente (32) aufweist.

4. Vorrichtung nach einem der vorhergehenden Ansprüche, **dadurch gekennzeichnet, dass** das Versteifungselement (32) aus Kunststoff gefertigt ist.

5. Vorrichtung nach einem der vorhergehenden Ansprüche, **dadurch gekennzeichnet, dass** das formelastische Material ein E-Modul von mehr als 600 N/mm² aufweist.

6. Vorrichtung nach einem der vorhergehenden Ansprüche, **dadurch gekennzeichnet, dass** das Versteifungselement (32) auswechselbar an dem Rückenelement (30) angeordnet ist, vorzugsweise in eine an dem Rückenelement (30) angeordnete Tasche eingeschoben ist.

7. Vorrichtung nach einem der vorhergehenden Ansprüche, **dadurch gekennzeichnet, dass** die Schulterbänder (40) in ihrer Länge verstellbar ausgebildet sind.

8. Vorrichtung nach einem der vorhergehenden Ansprüche, **dadurch gekennzeichnet, dass** die Schulterbänder (40) jeweils mit einem Ende (41) an dem Rückenelement (30) und mit dem anderen Ende (42) entweder an dem Rückenelement (30) oder dem Hüftelement (20) angeordnet sind.

9. Vorrichtung nach einem der vorhergehenden Ansprüche, **dadurch gekennzeichnet, dass** das Hüftelement (20) als vorzugsweise in der Weite verstellbarer Hüftgurt ausgebildet ist.

10. Vorrichtung nach einem der vorhergehenden Ansprüche, **dadurch gekennzeichnet, dass** das Elastomer ein E-Modul von weniger als 600 N/mm² aufweist.

11. Vorrichtung nach einem der vorhergehenden Ansprüche, **dadurch gekennzeichnet, dass** jedes der Beinbänder (50) lösbar an dem Hüftelement (20) befestigt ist.

12. Vorrichtung nach einem der vorhergehenden Ansprüche, **dadurch gekennzeichnet, dass** jedes der Beinbänder (50) in der Länge verstellbar ausgebildet ist.

13. Vorrichtung nach Anspruch 12,
**dadurch gekennzeichnet, dass** die obere Knieschlaufe (71) und/oder die untere Knieschlaufe (72) öffenbar und wiederverschließbar, vorzugsweise mittels eines Klettverschlusses oder eines sonstigen Verschlusses (74, 75), ausgebildet sind.

14. Vorrichtung nach einem der vorhergehenden Ansprüche, **dadurch gekennzeichnet, dass** jedes der Beinbänder (50) ein vorzugsweise lösbar befestigtes Fußelement (60) aufweist, welches eine Knöchelschlaufe (61) und eine Sohlenschlaufe (62) aufweist, welche insbesondere derart miteinander verbunden sind, dass sie ein 8-förmiges Element bilden.

15. Vorrichtung nach Anspruch 14,
**dadurch gekennzeichnet, dass** zwischen der Knöchelschlaufe (61) und der Sohlenschlaufe (62) ein Fersenband (63) angeordnet ist, welches vorzugsweise an ihren einander abgewandten Bereichen befestigt ist.

16. Vorrichtung nach einem der vorhergehenden Ansprüche, **dadurch gekennzeichnet, dass** die Beinbänder (50) in dem Bereich zwischen dem Knieelement (70) und dem Fußelement (60) in ihrer Länge verstellbar ausgebildet sind.

## Claims

1. Device (10) for assisting movements and lifting movements of the human body, which can be worn on the body, having a waist element (20), having two shoulder straps (40), having a back element (30) which is arranged on the waist element (20) and comprises at least one reinforcing element (32) which is produced at least in portions from a shape-elastic material, and having two leg straps (50) which are arranged on the waist element (20) and are produced at least in portions from an elastomer and are arranged on the waist element (20) in such a way that the extend in the extension of the back element (30), wherein each of the leg straps (50) comprises a knee element (70) which comprises an upper knee loop (71), a lower knee loop (72) and a kneecap element (73) that preferably leaves the kneecap free,
**characterised in that** the leg straps extend forwards from the back, over the knee.

2. Device according to claim 1,
**characterised in that** the reinforcing element (32) is oriented substantially in parallel with the spine in the state where the device (10) is applied on the body.

3. Device according to either of the preceding claims, **characterised in that** the back element (30) comprises two or more reinforcing elements (32).

4. Device according to any of the preceding claims, **characterised in that** the reinforcing element (32) is produced from plastics material.

5. Device according to any of the preceding claims, **characterised in that** the shape-elastic material has an E-modulus of more than 600 N/mm².

6. Device according to any of the preceding claims, **characterised in that** the reinforcing element (32) is arranged on the back element (30) in an exchangeable manner, preferably inserted into a pocket arranged on the back element (30).

7. Device according to any of the preceding claims, **characterised in that** the shoulder straps (40) are configured to be adjustable in length.

8. Device according to any of the preceding claims, **characterised in that** the shoulder straps (40) are in each case arranged having one end (41) on the back element (30) and having the other end (42) either on the back element (30) or the waist element (20).

9. Device according to any of the preceding claims, **characterised in that** the waist element (20) is configured as a waist belt that is adjustable in width.

10. Device according to any of the preceding claims, **characterised in that** the elastomer has an E-modulus of less than 600 N/mm².

11. Device according to any of the preceding claims, **characterised in that** each of the leg straps (50) is detachably fastened to the waist element (20).

12. Device according to any of the preceding claims, **characterised in that** each of the leg straps (50) is configured to be adjustable in length.

13. Device according to claim 12,
**characterised in that** the upper knee loop (71) and/or the lower knee loop (72) are configured such that they can be opened and closed again, preferably by means of a hook and loop fastener or another fastener (74, 75).

14. Device according to any of the preceding claims, **characterised in that** each of the leg straps (50) comprises a preferably detachably fastened foot element (60) which comprises an ankle loop (61) and a sole loop (62), which are in particular connected to one another in such a way that they form an element in the shape of a figure 8.

15. Device according to claim 14,
**characterised in that** a heel strap (63) is arranged between the ankle loop (61) and the sole loop (62), which heel strap is preferably fastened to their regions facing away from one another.

16. Device according to any of the preceding claims, **characterised in that** the leg straps (50) are configured to be adjustable in length in the region between the knee element (70) and the foot element (60).

## Revendications

1. Dispositif (10) pour venir à l'appui de mouvements et de mouvements de levée du corps humain, qui peut être porté sur le corps, comprenant un élément (20) de hanche, comprenant deux bandes (40) d'épaule, comprenant un élément (30) de dos, qui est monté sur l'élément (20) de hanche et qui a au moins un élément (32) de renforcement lequel est au moins par endroit en un matériau ayant de l'élasticité de forme, et comprenant deux jambières (50), qui sont montés sur l'élément (20) de hanche, qui sont au moins en un élastomère et qui sont montés sur l'élément (20) de hanche de manière à s'étendre dans le prolongement de l'élément (30) de dos, dans lequel chacune des jambières (50) a un élément (70) de genou, qui a une boucle (71) supérieure de genou, une boucle (72) inférieure de genou et un élément (73) de rotule laissant libre de préférence la rotule,
**caractérisé en ce que** les jambières s'étendent du dos vers l'avant sur le genou.

2. Dispositif selon la revendication 1,
**caractérisé en ce que** l'élément (32) de renforcement est, lorsque le dispositif (10) est à l'état mis sur le corps, dirigé sensiblement parallèlement à la colonne vertébrale.

3. Dispositif selon l'une des revendications précédentes, **caractérisé en ce que** l'élément (30) de dos a deux ou plusieurs éléments (32) de renforcement.

4. Dispositif selon l'une des revendications précédentes, **caractérisé en ce que** l'élément (32) de renforcement est en matière plastique.

5. Dispositif selon l'une des revendications précédentes, **caractérisé en ce que** le matériau à élasticité de forme a un module E de plus de 600 N/mm².

6. Dispositif selon l'une des revendications précédentes, **caractérisé en ce que** l'élément (32) de renforcement est monté sur l'élément (30) de dos de manière à pouvoir être remplacé, en étant de préférence glissé dans une poche disposée sur l'élément (30) de dos.

7. Dispositif selon l'une des revendications précédentes, **caractérisé en ce que** les bandes (40) d'épaule sont de longueur réglable.

8. Dispositif selon l'une des revendications précédentes, **caractérisé en ce que** les bandes (40) d'épaule sont montées respectivement par une extrémité (41) sur l'élément (30) de dos et par l'autre extrémité (42) soit sur l'élément (30) de dos, soit sur l'élément (20) de hanche.

9. Dispositif selon l'une des revendications précédentes, **caractérisé en ce que** l'élément (20) de hanche est constitué de préférence sous la forme d'une ceinture de hanche réglable en largeur.

10. Dispositif selon l'une des revendications précédentes, **caractérisé en ce que** l'élastomère a un module E de moins de 600 N/mm².

11. Dispositif selon l'une des revendications précédentes, **caractérisé en ce que** chacune des jambières (50) est fixée de manière amovible à l'élément (20) de hanche.

12. Dispositif selon l'une des revendications précédentes, **caractérisé en ce que** chacune des jambières (50) est de longueur réglable.

13. Dispositif selon la revendication 12,
**caractérisé en ce que** la boucle (71) supérieure de genou et/ou la boucle (72) inférieure de genou peut être ouverte et peut être refermée, de préférence au moyen d'une fermeture auto-agrippante ou d'une autre fermeture (74, 75).

14. Dispositif suivant l'une des revendications précédentes, **caractérisé en ce que** chacune des jambières (50) a un élément (60) de pied fixé de préférence de manière amovible, qui a une boucle (61) de cheville et une boucle (62) de plante, lesquelles sont en particulier reliées l'une à l'autre de manière à former un élément en forme de 8.

15. Dispositif suivant la revendication 14,
**caractérisé en ce qu'**entre la boucle (61) de cheville et la boucle (62) de plante est disposée une bande (63) de talon, qui est fixée de préférence à ses parties non tournées l'une vers l'autre.

16. Dispositif suivant l'une des revendications précédentes, **caractérisé en ce que** les jambières (50) sont, dans la partie comprise entre l'élément (70) de genou et l'élément (60) de pied, réglable en longueur.
